# EUROPEAN PATENT SPECIFICATION

(11) **EP 4 106 682 B1**
(45) Date of publication and mention of the grant of the patent: **30.09.2026**
(21) Application number: 21756965.6
(22) Date of filing: 16.02.2021
(51) Int. Cl.: A61F 2/72, A61F 2/68, A61H 3/00, A61B 5/11, A61B 5/00, A61F 2/60

(54) **METHOD FOR DETECTING INTENT IN AN ADAPTIVE LOWER LIMB DEVICE**
VERFAHREN ZUR ERKENNUNG EINER ABSICHT IN EINER ADAPTIVEN VORRICHTUNG DER UNTEREN GLIEDMASSEN
PROCÉDÉ DE DÉTECTION D'INTENTION DANS UN DISPOSITIF DE MEMBRE INFÉRIEUR ADAPTATIF

(30) Priority: 19.02.2020 US 202062978670 P
(43) Date of publication of application: 28.12.2022
(73) Proprietor: Vanderbilt University, Nashville, TN 37240 (US)
(72) Inventor: GOLDFARB, Michael, Nashville, Tennessee 37240 (US); LEE, Jantzen T., Nashville, Tennessee 37240 (US)
(74) Representative: Algemeen Octrooi- en Merkenbureau B.V.
(86) International application number: PCT/US2021/018246
(87) International publication number: WO 2021/167907

(56) References cited:
- US-A1- 2014 088 729
- US-A1- 2014 088 729
- US-A1- 2015 173 993
- US-A1- 2019 060 156
- US-A1- 2019 060 156
- US-B2- 10 499 851
- US-B2- 9 108 060

## Description

### FIELD OF THE INVENTION

This application describes methods to detect user intent for purposes of transitioning between various activities when using an adaptive lower limb device.

### BACKGROUND OF THE INVENTION

As prostheses and orthoses become increasingly electronically-controlled, they have an increasingly wider range of functional capabilities. In particular, these prostheses and orthoses are capable of adapting between multiple discrete activities, such as level walking, sloped walking, and stair ascent or descent. In such multifunctional devices, a more seamless transition between appropriate dedicated behaviors for these varying activities is desired. The existing devices often do not transition between discrete activities in a smooth fashion. The transition between discrete activities of a user in these existing devices is often too late or unreliable over time.

United Staes patent application US 2019/060156 A1 discloses a computer implemented method of semi-supervised intent recognition for an exoskeleton system. In one aspect, the method includes, in response to a state transition intention input, changing the exoskeleton system from operating in a first mode with sensitivity to detecting state transitions at a first sensitivity level to operating in a second mode with sensitivity to detecting state transitions at a second sensitivity level that is more sensitive than the first sensitivity level; identifying a state transition while operating in the second mode and using the second sensitivity level; and facilitating the identified state transition by actuating the exoskeleton system.

It would be desirable to have a device that more smoothly transitions between these discrete activities.

### SUMMARY OF THE INVENTION

The term embodiment and like terms are intended to refer broadly to all of the subject matter of this disclosure and the claims below. Statements containing these terms should be understood not to limit the subject matter described herein or to limit the meaning or scope of the claims below. Embodiments of the present disclosure covered herein are defined by the claims below, not this summary. This summary is a high-level overview of various aspects of the disclosure and introduces some of the concepts that are further described in the Detailed Description section below. This summary is not intended to identify key or essential features of the claimed subject matter. This summary is also not intended to be used in isolation to determine the scope of the claimed subject matter. The subject matter should be understood by reference to appropriate portions of the entire specification of this disclosure, any or all drawings and each claim.

According to a first aspect, the present invention is related to a method according to claim 1.

According to a second aspect, the present invention is related to a device according to claim 4.

### BRIEF DESCRIPTION OF THE DRAWINGS

The disclosure, and its advantages and drawings, will be better understood from the following description of exemplary embodiments together with reference to the accompanying drawings. These drawings depict only exemplary embodiments, and are therefore not to be considered as limitations on the scope of the various embodiments or claims.
FIG. 1 is an adaptive lower limb device according to the invention.
FIG. 2 is a schematic depicting a user transitioning from walking on a general flat surface to descending down stairs according to the invention.
FIG. 3 is a schematic depicting a user transitioning from walking on a general flat surface to ascending up stairs according to the present invention.
FIG. 4 is a schematic depicting a user transitioning from walking on a general flat surface to descending down stairs, which, however, is not part of the present invention.
FIG. 5 is a schematic depicting a user transitioning from sitting in a chair to standing up, which, however, is not part of the present invention.
FIG. 6 is a schematic depicting a plurality of activity controllers according to the present invention.

While the invention is susceptible to various modifications and alternative forms, specific implementations have been shown by way of example in the drawings and will be described in further detail herein. It should be understood, however, that the invention is not intended to be limited to the particular forms disclosed. Rather, the invention is to cover all modifications, equivalents, and alternatives falling within the scope of the invention as defined by the appended claims.

### DETAILED DESCRIPTION

According to one method, a user's intent is detected in an adaptive lower limb device. The adaptive lower limb device includes a device control unit. The device control unit includes a plurality of activity controllers and at least one accelerometer. The at least one accelerometer may be located within an inertial measurement sensor. The measured acceleration features are determined whether to correspond to a tapping movement initiated by a user with an intent to switch from a first one of the plurality of activity controllers to a second one of the plurality of two activity controllers. If the measured accelerating features correspond to the tapping movement, the device control unit of the adaptive lower limb device is switched from the first one of the plurality of activity controllers to the second one of the plurality of activity controllers.

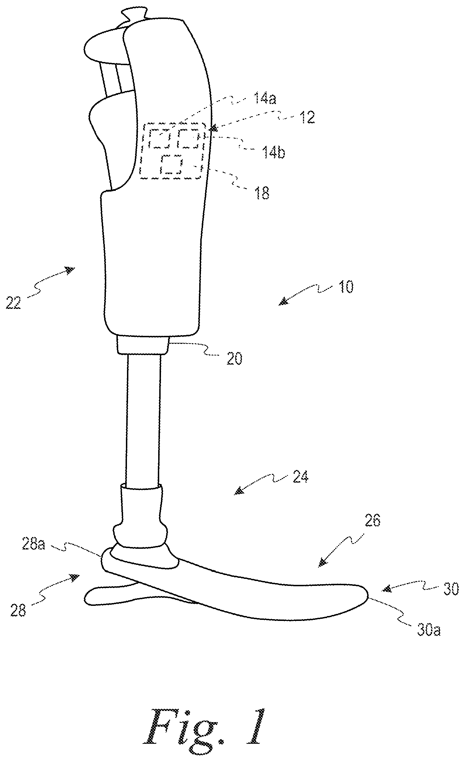

One non-limiting example of an adaptive lower limb device that may be used is shown in FIG. 1. The adaptive lower limb device may be used in the form of a prosthesis or an orthosis. The adaptive lower limb device of the present invention facilitates the gait of a user and, more specifically, improves the transition between discrete activities or tasks.

Referring still to FIG. 1, an adaptive lower limb device 10 is shown that assists a user in discrete activities or tasks such as level walking, sloped walking, standing, sitting, running, cycling, stair ascent and decent, and a locked knee mode. The adaptive lower limb device 10 includes a device control unit 12. The device control unit 12 includes a plurality of activity or task controllers 14a, 14b, and at least one accelerometer 18. The adaptive lower limb device 10 in one embodiment may include a load cell 20. The adaptive lower limb device 10 includes an upper section 22 and a lower section 24. The upper section 22 includes the device control unit 12 in this embodiment. The lower section 24 of the adaptive lower limb device 10 includes a foot area 26. The foot area 26 includes a heel portion 28 and a toe portion 30. The heel portion 28 includes a posterior portion 28a, while the toe portion 30 includes an anterior portion 30a.

The plurality of activity controllers 14a, 14b assists a user with a specific discrete activity. For example, an activity controller may be a walking controller, a sloped walking controller, a standing controller, a sitting controller, a running controller, a cycling controller, a stair ascent controller, and stair descent controller, and a locked knee controller. It is contemplated that the device control unit may include two or more of any of the combinations of activity controllers.

It is contemplated that the plurality of activity controllers may include two or more of the above identified activity controllers. It is contemplated that the device control unit of the adaptive lower limb device may include other activity controllers.

The activity controller and control methodology provides for stable and reliable interaction between the user and adaptive lower limb device. Control has been found to enable the user to interact with the adaptive lower limb device by leveraging its dynamics in a manner similar to normal gait, and also generates more stable and more predictable behavior. Non-limiting examples of selected controllers are described in U.S. Patent No. 8,986,396.

The at least one accelerometer 18 in the device control unit of the adaptive lower limb device assists in measuring the acceleration features. The measured acceleration features are used to determine whether a tapping movement has been initiated by a user with an intent to switch between activity controllers.

The at least one accelerometer may be located within an inertial measurement sensor (IMU). A typically IMU includes at least one accelerometer and at least one gyroscope. The IMU may be a multi-axis IMU in one embodiment. One non-limiting example of a multi-axis IMU includes a plurality of accelerometers and a plurality of gyroscopes.

Non-limiting examples of accelerometers or IMUs that may be used in the device control unit of the adaptive lower limb device include TDK's InvenSense MPU-6000 or TDK's InvenSense ICM-20948 IMUs. It is contemplated that other accelerometers may be used in the device control unit of the adaptive lower limb device.

The load cell 20 in the adaptive lower limb device, if used, assists in determining loads on the adaptive lower limb device 10. The load cell 20 is a device that measures forces. By determining the load of the adaptive lower limb device, a load cell can assist in distinguishing between events such as a typical heel strike or toe strike and a heel or toe strike acceleration signal. Load cells may be purchased for use or may be custom designed and integrated with the adaptive lower limb device. A non-limiting example of a load cell that may be used in the adaptive lower limb device is described in U.S. Patent No. 10,111,762. It is contemplated that other load cells may be used in the adaptive lower limb device.

The adaptive lower limb device (e.g., adaptive lower limb device 10) may be used in the methods of the present invention to infer intent of the user by the tapping motion. The tapping motion can be measured by the adaptive lower limb device via the at least one accelerometer 18 mounted in or on the adaptive lower limb device 10. The tapping may be a lighter tapping or may be a stronger tapping that might be referred to a slap, jab, poke or a punch. A tapping motion provides several advantages in the methods of the present invention. First, the tapping motion can occur anywhere on the adaptive lower limb device, according to the invention at the posterior portion of a heel device and/or at the anterior portion of a heel, and still be measured by the at least one accelerometer (e.g., the accelerometer 18) located anywhere on the adaptive lower limb device. Second, the tapping motion can be performed either by tapping on the adaptive lower limb device (e.g., with one's hand or fingers), or by tapping the adaptive lower limb device against the environment, which gives considerable flexibility when performing a volitional cue. Third, a plurality of successive taps may in another method be used, for example, to encode different information than a single tap motion, or to make a given volitional cue more strongly intentional.

The tapping motion may be performed by a posterior portion of a heel of an adaptive lower limb device (e.g., adaptive lower limb device 10). The tapping may be, for example, a single-tap motion, or a plurality of tapping motions. The plurality of tapping motions may be a double-tap motion or a triple-tap motion. The tapping of the posterior portion of the heel of an adaptive lower device in one method is performed against a riser of a stair, which indicates the intent of a user to perform a stair descent. The resulting "heel strike" acceleration signal can be disambiguated from a typical heel strike by the absence of load. The absence of a load may be determined in embodiments wherein the adaptive lower limb device includes a load cell (e.g., the load cell 20). The load cell is configured to measure loads and assists in distinguishing between a typical heel strike and a heel strike acceleration signal.

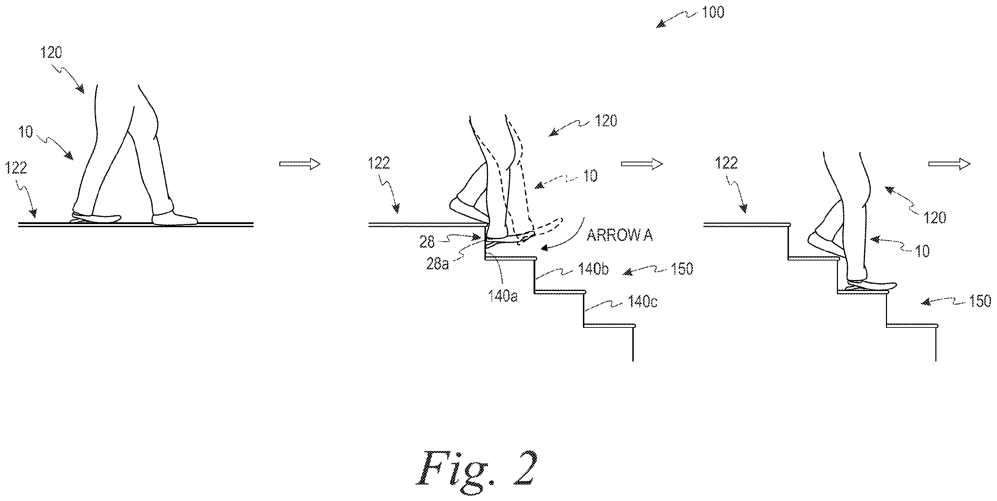

One non-limiting example of this method is depicted in FIG. 2. FIG. 2 depicts a method 100 that includes a user or individual 120 walking on a generally flat surface 122. At this point, the activity controller of the adaptive lower limb device 10 is being used as a walking controller. The user 120 then taps the posterior portion 28a of his or her heel 28 against one of a plurality of stair risers 140a-140c. The user 120 will typically select a riser closest to the generally flat surface 122. In FIG. 2, for example, the user 120 taps the stair riser 140a (in the direction of Arrow A) with the posterior portion 28a of his or her heel 28. After the user 120 taps the stair riser 140a, the activity controller in the device control unit 12 is switched from the walking controller to the stair decent controller. This occurs before the user 120 continues his or her descent down stairs 150.

Alternatively or additionally, tapping may be performed by an anterior portion of a toe of an adaptive lower limb device (e.g., adaptive lower limb device 10). The tapping may be, for example, a single-tap motion, or a plurality of tapping motions. The plurality of tapping motions may be a double-tap motion or a triple-tap motion. The tapping of the anterior portion of the toe of an adaptive lower device in one method is performed against a riser of a stair, which indicates the intent of a user to perform a stair ascent. As with the heel tap signal discussed above, the resulting acceleration from this signal can be disambiguated form a standard toe strike via the absence of load. The absence of a load may be determined in embodiments wherein the adaptive lower limb includes a load cell (e.g., load cell 20). The load cell is configured to measure loads and assists in distinguishing between a typical toe strike and a toe strike acceleration signal. In addition to the presence or absence of a load, tapping in other circumstances can be disambiguated from other events via other measurements, such as limb orientation in space or limb angular velocity through space.

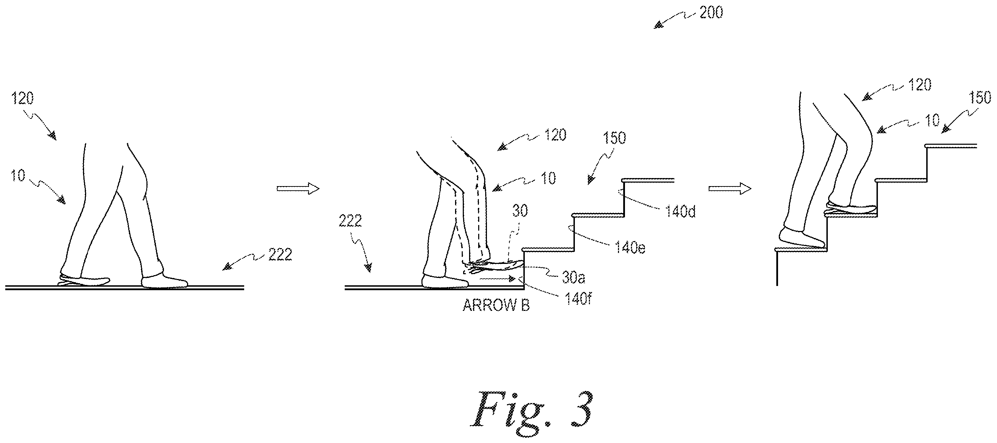

One non-limiting example of this method is depicted in FIG. 3. FIG. 3 depicts a method 200 that includes the user or individual 120 walking on a generally flat surface 222. At this point, the activity controller of the adaptive lower limb device 10 is being used as a walking controller. The user 120 then taps the anterior portion 30a of his or her toe 30 (in the direction of Arrow B) against one of a plurality of stair risers 140d-140f. The user 120 will typically select a riser closest to the generally flat surface 222. In FIG. 3, for example, the user 120 taps the stair riser 140f with the anterior portion 30a of his or her toe 30. After the user 120 taps the stair riser 140f, the activity controller in the device control unit 12 is switched from the walking controller to the stair ascent controller. This occurs before the user 120 continues his or her ascent up the stairs 150.

In a further method, which, however, is not part of the present invention, the user can convey intent to change activity by tapping the adaptive lower limb device in one method with, for example, his or her arm or hand. It is noted that since vibration travels through a limb, the user need not direct tap on the adaptive lower limb device itself. In another method, the user can convey intent to change activity by tapping his or her leg that includes the adaptive lower limb device with, for example, his or her arm or hand. The at least one accelerometer is mounted on the adaptive lower limb device will detect the vibration resulting from the tap or slap, regardless of whether the user taps or slaps the adaptive lower limb device, or the limb to which the device is attached.

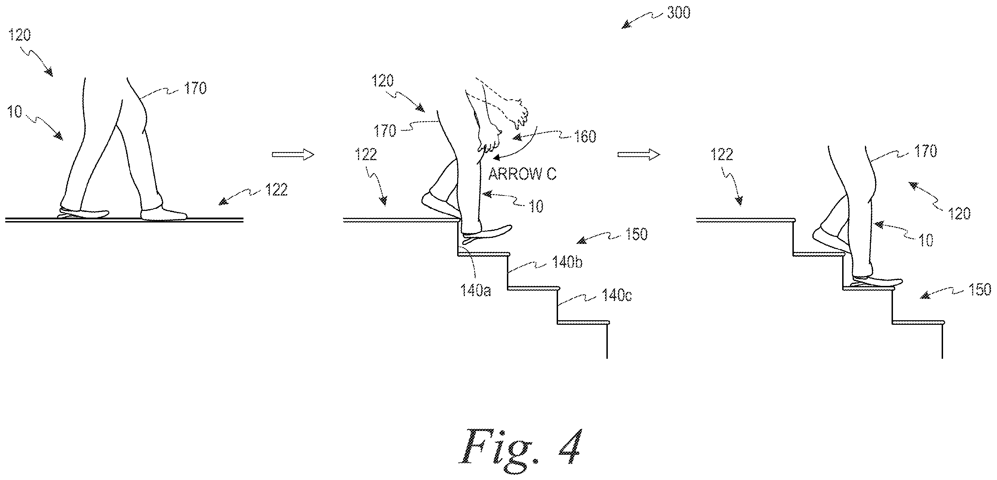

One non-limiting example of this method is depicted in FIG. 4. FIG. 4 depicts a method 300 that includes the user or individual 120 walking on the generally flat surface 122. At this point, the activity controller of the adaptive lower limb device 10 is being used as a walking controller. The user 120 then taps the adaptive lower limb device 10 or his or her leg (e.g., the thigh) with an arm or a hand. In this specific example, the user 120 taps leg 170 with his or her hand 160. This motion shown in FIG. 4 with the hand 160 moving in the generally direction of arrow C. After the user 120 taps the leg 170 with his or her hand 160, the activity controller in the device control unit 12 is switched from the walking controller to the stair decent controller. This occurs before the user continues his or her descent down the stairs 150.

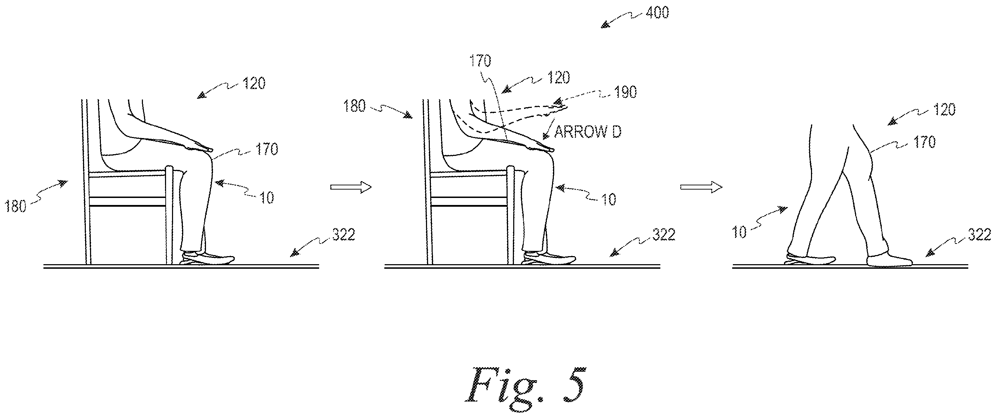

Another non-limiting example of this method is depicting in FIG. 5. FIG. 5 depicts a method 400 that includes the user or individual 120 sitting in a chair 180. At this point, the activity controller of the adaptive lower limb device 10 is being used as a sitting controller. The user 120 then taps the adaptive lower limb device 10 or his or her leg (e.g., the thigh) with an arm or a hand. In this specific example, the user 120 taps the leg 170 with his or her arm 190 or hand 160. This motion is shown in FIG. 5 with the arm or hand 160 moving in the generally direction of arrow D. After the user 120 taps the adaptive lower limb device 10 with his or her arm 190 or hand 160, the activity controller in the device control unit 12 is switched from the walking controller to the standing controller. This occurs before the user transitions from the sitting position to the standing position on a generally flat surface 322.

It is contemplated in another method which, however, is not part of the present invention, that the user may tap the adaptive lower limb device or his or her leg with something other than an arm or hand. For example, a user may tap the adaptive lower limb device or his or her leg with another object, or vice versa. The tapping may be, for example, a single-tap motion, or a plurality of tapping motions. The plurality of tapping motions may be a double-tap motion or a triple-tap motion.

A user can use a single tap, double tap, or triple tap to indicate intent to perform various activities. For example, in one method, a double-tap motion (i.e., two taps in quick succession) indicates an intent to transition from walking to a stair ascent. In a further method, a triple-tap motion indicates an intent to transition from sitting to standing. It is contemplated that the plurality of tapping motions may indicate other transitions between the activity controllers.

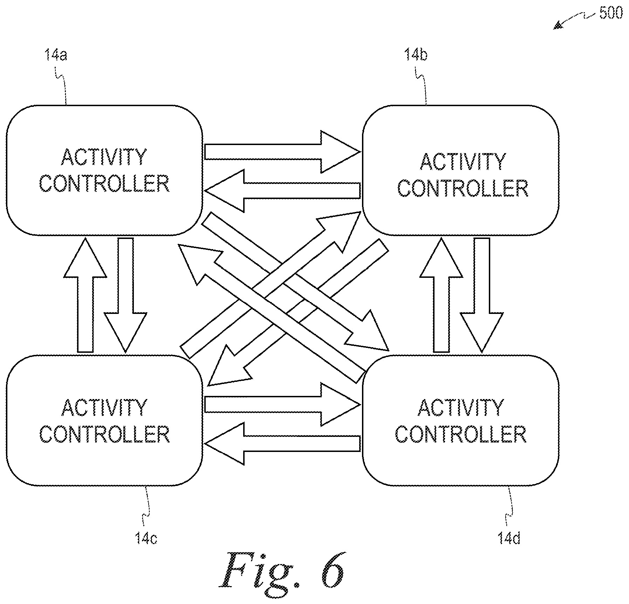

Referring to FIG. 6, a schematic 500 is shown with first, second, third and fourth activity controllers 14a-14d. Each of the activity controllers 14a-14d can communicate directly with each other. It is contemplated that the number of activity controllers can vary in the device control unit in the adaptive lower limb device. For example, the number of activity controllers may be exactly two activity controllers or a higher number of activity controllers such as 3, 4, 5, 6 or more activity controllers.

These adaptive lower limb devices of the present invention and the methods of using the same are applicable with lower limb prostheses and orthoses controlled by microprocessors. This application is associated with a knee prosthesis. It is contemplated that it may be used with other technologies such as robotic ankles, knees or lower limb orthoses. This invention is advantageous in that intent is inferred without requiring awkward or unnatural movements. It would also be desirable for a user to provide an intentional act that does not require an undue effort by the user to assist the adaptive lower limb device in more smoothly transitioning. The cues are desirably subtle and relative to the natural movement associated with the current activity.

The foregoing description of the embodiments, including illustrated embodiments, has been presented only for the purpose of illustration and description and is not intended to be exhaustive or limiting to the precise forms disclosed. Numerous modifications, adaptations, and uses thereof will be apparent to those skilled in the art.

## Claims

1. A method for detecting a user's intent in an adaptive lower limb device (10), the method comprising:
providing the adaptive lower limb device (10) including a device control unit (12), the device control unit including a plurality of activity controllers (14a, 14b) and at least one accelerometer (18);
measuring acceleration features via the at least one accelerometer (18); and
determining whether the measured acceleration features correspond to a tapping movement initiated by a user with an intent to switch from a first one of the plurality of activity controllers to a second one of the plurality of activity controllers; and
if the measured accelerating features correspond to the tapping movement, switching the control unit of the adaptive lower limb device (10) from the first one of the plurality of activity controllers to the second one of the plurality of activity controllers,
**characterized in that**
the tapping movement initiated by a user is tapping of a posterior portion (28a) of a heel (28) against a stair riser (140a-140c), wherein the first one of the plurality of activity controllers (14a, 14b) is a walking controller and the second one of the plurality of activity controllers is a stair descent controller, and tapping the posterior portion of the heel against the stair riser switches the device control unit from a walking controller to a stair descent controller; and/or
the tapping movement initiated by a user is tapping of an anterior portion (30a) of the toe (30) against a stair riser (140d-140f), wherein the first one of the plurality of activity controllers (14a, 14b) is a walking controller and the second one of the plurality of activity controllers is a stair ascent controller, and tapping the anterior portion of the toe against the stair riser switches the device control unit from a walking controller to a stair ascent controller.

2. The method of claim 1, wherein the tapping motion is a single-tap motion or a plurality of tapping motions.

3. The method of claim 1, wherein the adaptive lower limb device further includes a load cell (20).

4. An adaptive lower limb device (10) for detecting a user's intent, the device comprising:
a device control unit (12) including a plurality of activity controllers (14a, 14b) and at least one accelerometer (18), the at least on accelerometer being configured to measure acceleration features,
wherein the acceleration features are configured to determine whether a tapping movement has been initiated by a user with an intent to switch from a first one of the plurality of activity controllers to a second one of the plurality of activity controllers; and
wherein if the accelerating features correspond to the tapping movement, the adaptive lower limb device (10) being configured to switch from the first one of the plurality of activity controllers to the second one of the plurality of activity controllers
**characterized in that**
the tapping movement initiated by a user is tapping of a posterior portion (28a) of a heel (28) against a stair riser (140a-140c), wherein the first one of the plurality of activity controllers (14a, 14b) is a walking controller and the second one of the plurality of activity controllers is a stair descent controller, and tapping the posterior portion of the heel against the stair riser switches the device control unit from a walking controller to a stair descent controller, and/or
the tapping movement initiated by a user is tapping of an anterior portion (30a) of the toe (30) against a stair riser (140d-140f), ), wherein the first one of the plurality of activity controllers (14a, 14b) is a walking controller and the second one of the plurality of activity controllers is a stair ascent controller, and tapping the anterior portion of the toe against the stair riser switches the device control unit from a walking controller to a stair ascent controller.

5. The device of claim 4 further including a load cell (20).

6. The method of claim 1, wherein the tapping movement initiated by a user:
is performed using a hand or an arm (160) of the user to tap against the adaptive lower limb device (10); or
is performed using a hand or an arm (160) of the user to tap the leg containing the adaptive lower limb device (10).

## Patentansprüche

1. Verfahren zur Erkennung einer Absicht eines Benutzers in einer adaptiven Vorrichtung (10) der unteren Gliedmaßen, wobei das Verfahren Folgendes umfasst:
Bereitstellen der adaptiven Vorrichtung (10) der unteren Gliedmaßen, die eine Vorrichtungssteuereinheit (12) beinhaltet, wobei die Vorrichtungssteuereinheit eine Vielzahl von Aktivitätssteuergeräten (14a, 14b) und mindestens einen Beschleunigungsmesser (18) beinhaltet;
Messen von Beschleunigungsmerkmalen über den mindestens einen Beschleunigungsmesser (18); und
Bestimmen, ob die gemessenen Beschleunigungsmerkmale einer Tippbewegung entsprechen, die von einem Benutzer mit einer Absicht eingeleitet wird, von einem ersten der Vielzahl von Aktivitätssteuergeräten zu einem zweiten der Vielzahl von Aktivitätssteuergeräten zu schalten; und
wenn die gemessenen Beschleunigungsmerkmale der Tippbewegung entsprechen, Schalten der Steuereinheit der adaptiven Vorrichtung (10) der unteren Gliedmaßen von dem ersten der Vielzahl von Aktivitätssteuergeräten auf den zweiten der Vielzahl von Aktivitätssteuergeräten,
**dadurch gekennzeichnet, dass**
die von einem Benutzer eingeleitete Tippbewegung ein Tippen eines hinteren Abschnitts (28a) einer Ferse (28) gegen einen senkrechten Teil (140a-140c) einer Treppe ist, wobei das erste der Vielzahl von Aktivitätssteuergeräten (14a, 14b) ein Gehsteuergerät ist und das zweite der Vielzahl von Aktivitätssteuergeräten ein Treppenabstiegsteuergerät ist und ein Tippen des hinteren Abschnitts der Ferse gegen den senkrechten Teil der Treppe die Vorrichtungssteuereinheit von einem Gehsteuergerät zu einem Treppenabstiegsteuergerät schaltet; und/oder
die von einem Benutzer eingeleitete Tippbewegung ein Tippen eines vorderen Abschnitts (30a) der Zehe (30) gegen einen senkrechten Teil (140d-140f) einer Treppe ist, wobei das erste der Vielzahl von Aktivitätssteuergeräten (14a, 14b) ein Gehsteuergerät ist und das zweite der Vielzahl von Aktivitätssteuergeräten ein Treppensteigsteuergerät ist und ein Tippen des vorderen Abschnitts der Zehe gegen den senkrechten Teil der Treppe die Vorrichtungssteuereinheit von einem Gehsteuergerät zu einem Treppensteigsteuergerät schaltet.

2. Verfahren nach Anspruch 1, wobei die Tippgeste eine Einzeltippgeste oder eine Vielzahl von Tippgesten ist.

3. Verfahren nach Anspruch 1, wobei die adaptive Vorrichtung der unteren Gliedmaßen ferner eine Wägezelle (20) beinhaltet.

4. Adaptive Vorrichtung (10) der unteren Gliedmaßen zur Erkennung einer Absicht eines Benutzers, wobei die Vorrichtung Folgendes umfasst:
eine Vorrichtungssteuereinheit (12), die eine Vielzahl von Aktivitätssteuergeräten (14a, 14b) und mindestens einen Beschleunigungsmesser (18) beinhaltet, wobei der mindestens eine Beschleunigungsmesser dazu konfiguriert ist, Beschleunigungsmerkmale zu messen,
wobei die Beschleunigungsmerkmale dazu konfiguriert sind, zu bestimmen, ob eine Tippbewegung von einem Benutzer mit einer Absicht eingeleitet worden ist, von einem ersten der Vielzahl von Aktivitätssteuergeräten zu einem zweiten der Vielzahl von Aktivitätssteuergeräten zu schalten; und
wobei, wenn die Beschleunigungsmerkmale der Tippbewegung entsprechen, die adaptive Vorrichtung (10) der unteren Gliedmaßen dazu konfiguriert ist, von dem ersten der Vielzahl von Aktivitätssteuergeräten zu dem zweiten der Vielzahl von Aktivitätssteuergeräten zu schalten,
**dadurch gekennzeichnet, dass**
die von einem Benutzer eingeleitete Tippbewegung ein Tippen eines hinteren Abschnitts (28a) einer Ferse (28) gegen einen senkrechten Teil (140a-140c) einer Treppe ist, wobei das erste der Vielzahl von Aktivitätssteuergeräten (14a, 14b) ein Gehsteuergerät ist und das zweite der Vielzahl von Aktivitätssteuergeräten ein Treppenabstiegsteuergerät ist und ein Tippen des hinteren Abschnitts der Ferse gegen den senkrechten Teil der Treppe die Vorrichtungssteuereinheit von einem Gehsteuergerät zu einem Treppenabstiegsteuergerät schaltet, und/oder
die von einem Benutzer eingeleitete Tippbewegung ein Tippen eines vorderen Abschnitts (30a) der Zehe (30) gegen einen senkrechten Teil (140d-140f) einer Treppe ist, wobei das erste der Vielzahl von Aktivitätssteuergeräten (14a, 14b) ein Gehsteuergerät ist und das zweite der Vielzahl von Aktivitätssteuergeräten ein Treppensteigsteuergerät ist und ein Tippen des vorderen Abschnitts der Zehe gegen den senkrechten Teil der Treppe die Vorrichtungssteuereinheit von einem Gehsteuergerät zu einem Treppensteigsteuergerät schaltet.

5. Vorrichtung nach Anspruch 4, ferner beinhaltend eine Wägezelle (20).

6. Verfahren nach Anspruch 1,
wobei die von einem Benutzer eingeleitete Tippbewegung:
unter Verwendung einer Hand oder eines Arms (160) des Benutzers durchgeführt wird, um gegen die adaptive Vorrichtung (10) der unteren Gliedmaßen zu tippen; oder
unter Verwendung einer Hand oder eines Arms (160) des Benutzers durchgeführt wird, um auf das Bein zu tippen, das die adaptive Vorrichtung (10) der unteren Gliedmaßen enthält.

## Revendications

1. Procédé de détection de l'intention d'un utilisateur dans un dispositif de membre inférieur adaptatif (10), le procédé comprenant :
la fourniture du dispositif de membre inférieur adaptatif (10) comprenant une unité de commande de dispositif (12), l'unité de commande de dispositif comprenant une pluralité de dispositifs de commande d'activité (14a, 14b) et au moins un accéléromètre (18) ;
la mesure des caractéristiques d'accélération par l'intermédiaire dudit au moins un accéléromètre (18) ; et
la détermination pour savoir si les caractéristiques d'accélération mesurées correspondent à un mouvement de tapotement initié par un utilisateur avec l'intention de passer d'un premier de la pluralité de dispositifs de commande d'activité à un second de la pluralité de dispositifs de commande d'activité ; et
si les caractéristiques d'accélération mesurées correspondent au mouvement de tapotement, le passage de l'unité de commande du dispositif de membre inférieur adaptatif (10) du premier de la pluralité de dispositifs de commande d'activité au second de la pluralité de dispositifs de commande d'activité,
**caractérisé en ce que**
le mouvement de tapotement initié par un utilisateur est le tapotement d'une partie postérieure (28a) d'un talon (28) contre une contremarche d'escalier (140a à 140c), ledit premier de la pluralité de dispositifs de commande d'activité (14a, 14b) étant un dispositif de commande de marche et ledit second de la pluralité de dispositifs de commande d'activité étant un dispositif de commande de descente d'escalier, et le tapotement de la partie postérieure du talon contre la contremarche d'escalier fait passer l'unité de commande de dispositif d'un dispositif de commande de marche à un dispositif de commande de descente d'escalier ; et/ou
le mouvement de tapotement initié par un utilisateur est le tapotement d'une partie antérieure (30a) de l'orteil (30) contre une contremarche d'escalier (140d à 140f), ledit premier de la pluralité de dispositifs de commande d'activité (14a, 14b) étant un dispositif de commande de marche et ledit second de la pluralité de dispositifs de commande d'activité étant un dispositif de commande de montée d'escalier, et le tapotement de la partie antérieure de l'orteil contre la contremarche d'escalier fait passer l'unité de commande de dispositif d'un dispositif de commande de marche à un dispositif de commande de montée d'escalier.

2. Procédé selon la revendication 1, ledit mouvement de tapotement étant un mouvement de tapotement unique ou une pluralité de mouvements de tapotement.

3. Procédé selon la revendication 1, ledit dispositif de membre inférieur adaptatif comprenant en outre une cellule de charge (20).

4. Dispositif de membre inférieur adaptatif (10) destiné à détecter l'intention d'un utilisateur, le dispositif comprenant :
une unité de commande de dispositif (12) comprenant une pluralité de dispositifs de commande d'activité (14a, 14b) et au moins un accéléromètre (18), ledit au moins un accéléromètre étant configuré pour mesurer des caractéristiques d'accélération,
lesdites caractéristiques d'accélération étant configurées pour déterminer si un mouvement de tapotement a été initié par un utilisateur avec l'intention de passer d'un premier de la pluralité de dispositifs de commande d'activité à un second de la pluralité de dispositifs de commande d'activité ; et
si lesdites caractéristiques d'accélération correspondent au mouvement de tapotement, ledit dispositif de membre inférieur adaptatif (10) étant configuré pour passer du premier dispositif de commande d'activité de la pluralité de dispositifs de commande d'activité au second dispositif de commande d'activité de la pluralité de dispositifs de commande d'activité,
**caractérisé en ce que**
le mouvement de tapotement initié par un utilisateur est le tapotement d'une partie postérieure (28a) d'un talon (28) contre une contremarche d'escalier (140a à 140c), ledit premier de la pluralité de dispositifs de commande d'activité (14a, 14b) étant un dispositif de commande de marche et ledit second de la pluralité de dispositifs de commande d'activité étant un dispositif de commande de descente d'escalier, et le tapotement de la partie postérieure du talon contre la contremarche d'escalier fait passer l'unité de commande de dispositif d'un dispositif de commande de marche à un dispositif de commande de descente d'escalier, et/ou
le mouvement de tapotement initié par un utilisateur est le tapotement d'une partie antérieure (30a) de l'orteil (30) contre une contremarche d'escalier (140d à 140f), ledit premier de la pluralité de dispositifs de commande d'activité (14a, 14b) étant un dispositif de commande de marche et ledit second de la pluralité de dispositifs de commande d'activité étant un dispositif de commande de montée d'escalier, et le tapotement de la partie antérieure de l'orteil contre la contremarche d'escalier fait passer l'unité de commande de dispositif d'un dispositif de commande de marche à un dispositif de commande de montée d'escalier.

5. Dispositif selon la revendication 4, comprenant en outre une cellule de charge (20).

6. Procédé selon la revendication 1,
ledit mouvement de tapotement initié par un utilisateur :
étant effectué à l'aide d'une main ou d'un bras (160) de l'utilisateur pour tapoter contre le dispositif de membre inférieur adaptatif (10) ; ou
étant effectué à l'aide d'une main ou d'un bras (160) de l'utilisateur pour tapoter la jambe contenant le dispositif de membre inférieur adaptatif (10).
